# EUROPEAN PATENT APPLICATION

(11) **EP 1 424 644 A1**
(43) Date of publication of application: **02.06.2004**
(21) Application number: 02741219.6
(22) Date of filing: 20.06.2002
(51) Int. Cl.: G06F 17/60

(54) **MEASUREMENT ASSISTING DEVICE AND MEASUREMENT INSTRUMENT USING THE SAME**

(30) Priority: 22.06.2001 JP 2001189862
(71) Applicant: ARKRAY, Inc., Kyoto 691-8045 (JP)
(72) Inventor: KAWATAHARA, Masanao, Kyoto-shi, Kyoto 601-8045 (JP)
(74) Representative: Piésold, Alexander J.
(86) International application number: PCT/JP2002/006171
(87) International publication number: WO 2003/001424

(57) **Abstract**

A measurement assisting system in which a point or points depending on the measurement result are given to the measurer, a reward depending on the obtained points are given to the measurer, and thus the measurer is motivated to make an accurate measurement. A patient sends data on the measurement made by using a blood sugar meter (1) to a self-measurement assisting server (6) through a game machine (2) with a communication function using a mobile telephone (3) over the Internet (4). The self-measurement assisting server (6) gives the patient a point or points corresponding to the evaluation of the measurement data. If the obtained points exceeds a predetermined value, the self-measurement assisting server (6) sends an electronic medium reward (on-line incentive) such as a game characteristic usable by the game machine (2) to the game machine (2) of the patient.

## Description

### Technical Field

The present invention relates to a measurement assisting system that motivates measurers to perform precise measurements or the like by giving points according to measurement results and giving the measurers rewards according to the obtained points.

### Background Art

Conventionally, portable measuring instruments for measuring blood sugar level have been developed for diabetics who need to control the blood sugar level routinely, and these instruments are used for self-measurement of blood sugar. Under the guidance of doctors, the diabetics measure the blood sugar level by themselves using the measuring instrument at a given time (e.g., before and after meals and at bedtime) everyday, and record the measured values. When undergoing a medical examination, the diabetics present the measured values that have been recorded to the doctors to receive the doctors' diagnosis or guidance on self-administration of the blood sugar level.

Further, in recent years, systems are known that enable patients to be examined by doctors or the like at home by transmitting measurement data to medical institutions via communication lines such as a phone line.

For effective treatment of diabetes, each patient is required to make an accurate self-measurement of blood sugar. For the purpose of providing motivation for making self-measurement of blood sugar, medical institutions or the like educate the patients so that they can fully recognize the importance of controlling blood sugar. However, child diabetics may not be able to understand the importance of blood sugar control, and accordingly it is difficult to motivate them effectively.

### Disclosure of Invention

To solve the above problems, it is an object of the present invention to provide a measurement assisting system that motivates measurers to perform precise measurements or the like by giving points according to measurement results and giving the measurers rewards according to the obtained points.

To achieve the above-described object, a measurement assisting device according to the present invention is to be connected to a measuring device and an electronic device of measurers via a communication medium. The measurement assisting device includes a point storage unit for storing an obtained point for each measurer, a point processing unit for adding a point given according to an evaluation of measurement data of a measurer received from the measuring device via the communication medium to the previously obtained points of the measurer stored in the point storage unit, an electronic medium reward storage unit for storing an electronic medium reward to be transmitted to the electronic device of the measurer via the communication medium and used therein, and a reward issuing unit for transmitting the electronic medium reward according to the obtained point of the measurer from the electronic medium reward storage unit to the electronic device of the measurer via the communication medium.

Examples of the electronic medium reward include a game program, a game character or item to be used in a game machine, music data to be used in an audio instrument, image data to be used in an electronic device with a display, such as a mobile phone, and electronic cash or a coupon that can be used by the measurer in payment in on-line shopping on the Internet or the like. However, the electronic medium reward is not restricted thereto.

As described above, points according to the evaluation of the measurement data obtained by the measuring device are given, and the electronic medium reward according to the obtained points is transmitted to the electronic device of the measurer, whereby the measurer is encouraged to make efforts to have a good evaluation on the measurement data. Therefore, it is possible to provide a measurement assisting device that motivates the measurer to perform a precise measurement or the like.

It is preferable that the measurement assisting device is to be connected to an information processing device of an administrative institution via the communication medium, and the measurement assisting device further includes a reception processing unit for receiving a point from the information processing device via the communication medium, the point being determined by an administrator in the administrative institution according to the evaluation of the measurement data and input to the information processing device.

In the case where the measuring device is a device for measuring, for example, items relating to the health condition of the measurer, the administrator may be a doctor, a public health nurse, a dietitian, an instructor of a fitness club, or the like, but is not restricted thereto. As described above, the administrator determines points according to the evaluation of the measurement data and transmits the determined points to the measurement assisting device from the information processing device used by the administrator. Thus, the points given to the measurer can reflect whether a precise measurement is performed according to the guidance of the administrator, or whether the effect of the guidance is seen in the measurement data.

Further, it is preferable that the measurement assisting device further includes a reference value storage unit for storing a standard for calculating points, and the point processing unit evaluates the measurement data received from the measuring device via the communication medium according to the standard stored in the reference value storage unit and determines a point to give to the measurer according to the evaluation.

According to this configuration, the standard for calculating points is previously set in the reference value storage unit so that points to be given are determined automatically by the point processing unit. Therefore, points can be given fairly, and without increasing the burden on the administrator besides.

In this configuration, the reference value storage unit may store a target measurement value, and the point processing unit may give higher points as an actual measurement value is closer to the target measurement value.

Alternatively, the reference value storage unit may store a predicted value input by the measurer before a measurement, and the point processing unit may give higher points as an actual measurement value is closer to the predicted value. This is advantageous in that the measurer is encouraged to perform earnest predictions of measurement values, particularly in the case where importance lies in measurer's capability of properly predicting measurement values, such as the case where a diabetic measures the blood sugar level.

Alternatively, the reference value storage unit may store a target value relating to measurement performing conditions, and the point processing unit may give higher points as a value relating to actual measurement performing conditions is closer to the target value. This is advantageous in that the measurer is motivated to perform a measurement properly at a given time, in the case where importance lies not only in the resulting measurement value but also in regular measurements to be performed according to the guidance of the administrator.

Further, in order to achieve the above-described object, a measuring device according to the present invention is to be connected to the measurement assisting device configured as any one of the above-described measurement assisting devices via a communication medium and transmit measurement data to the measurement assisting device via the communication medium. A point is given according to an evaluation of the measurement data in the measurement assisting device, and an electronic medium reward to be used in an electronic device of a measurer is supplied according to a cumulative total of the point.

Therefore, it is possible to provide a measuring device capable of motivating a measurer to perform a precise measurement by the electronic medium reward given to the measurer according to the evaluation of the measurement data.

Furthermore, in order to achieve the above-described object, a program according to the present invention is to be read in a computer to be connected to a measuring device and an electronic device of measurers via a communication medium. The computer includes a point storage unit for storing an obtained point for each measurer and an electronic medium reward storage unit for storing an electronic medium reward to be transmitted to the electronic device of the measurers via the communication medium and used therein. The program makes the computer execute a point processing of adding a point given according to an evaluation of measurement data of a measurer received from the measuring device via the communication medium to the previously obtained points of the measurer stored in the point storage unit, and a reward issuance processing of transmitting the electronic medium reward according to the obtained point of the measurer from the electronic medium reward storage unit to the electronic device of the measurer via the communication medium.

When the computer is made to read and execute the program, the measurement assisting device according to the present invention can be realized by this computer.

### Brief Description of Drawings

Figure 1 is a schematic diagram illustrating the configuration of a measurement assisting system according to Embodiment 1 of the present invention.
Figure 2 is a block diagram illustrating the configuration of a server that functions as a measurement assisting device in the measurement assisting system.
Figure 3 is a flowchart illustrating the operations of respective devices included in the measurement assisting system.
Figure 4 is a block diagram illustrating the configuration of a server that functions as a measurement assisting device in a measurement assisting system according to Embodiment 2 of the present invention.
Figure 5 is a graph showing an example of the relationship between a target value set in relation to point supply and an actual measurement value.

### Best Mode for Carrying Out the Invention

### (Embodiment 1)

Hereinafter, one embodiment of the present invention will be described with reference to the drawings.

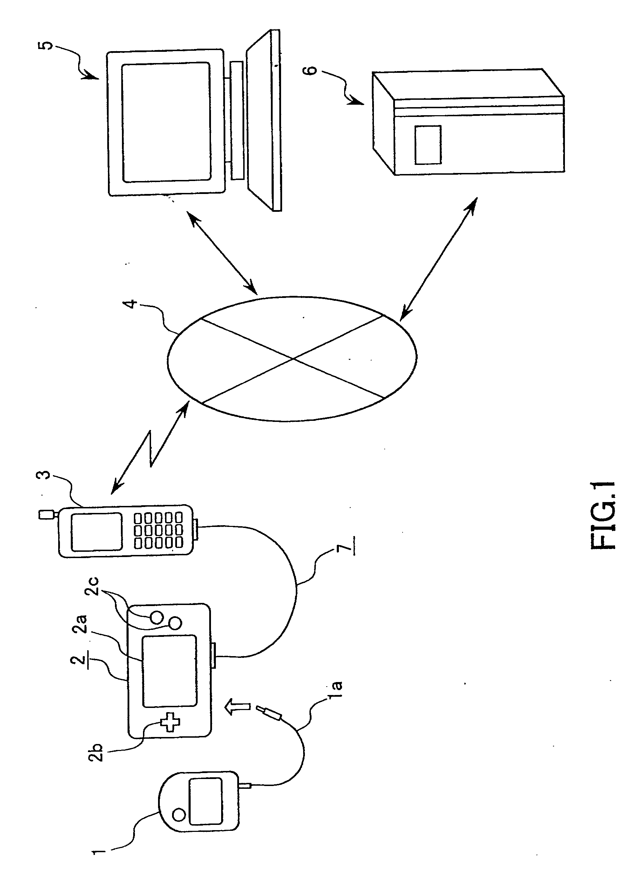

Initially, the overall configuration of a measurement assisting system according to one embodiment of the present invention will be described schematically with reference to Figure 1.

In Figure 1, numeral 1 denotes a blood sugar meter (measuring device) of a diabetic (hereinafter, merely referred to as a "patient"), numeral 2 denotes a game machine (electronic device), numeral 3 denotes a mobile phone, and numeral 4 denotes the Internet (communication medium). Further, numeral 5 denotes a computer of a medical institution where the patient is treated (information processing device of an administrative institution). Numeral 6 denotes a computer located in a data center of a provider of this measurement assisting system, which serves as a self-measurement assisting server (measurement assisting device) for collecting measurement data for each patient, managing a point given according to the evaluation of the measurement data, and giving a reward to the patient who has achieved the number of points satisfying a predetermined condition.

The blood sugar meter 1 measures the blood sugar level electrically or optically using a disposable sensor to which the diabetic attaches a slight amount of blood collected from a finger tip or the like. The measured blood sugar level and other pieces of information such as the time at which the measurement was performed are stored as measurement data in a memory in the blood sugar meter 1, and are transmitted to the self-measurement assisting server 6 as described later.

The game machine 2 is provided with a liquid crystal display screen 2a and operating buttons 2b and 2c. By loading a cartridge (not shown) as a recording medium storing a game program into this game machine, various kinds of games can be played.

Further, the game machine 2 can be connected to the mobile phone 3 via a communication cable 7, and when a cartridge storing a communication control program is loaded, access to the Internet 4 is allowed via the mobile phone 3. Thus, the user of the game machine 2 can, for example, download a new game program, a game character, and the like from a site administrated by the maker of the game machine 2, or play a game with other users. Further, as described later, it is also possible to download a game character or the like as an on-line incentive from the self-measurement assisting server 6 by accessing the same.

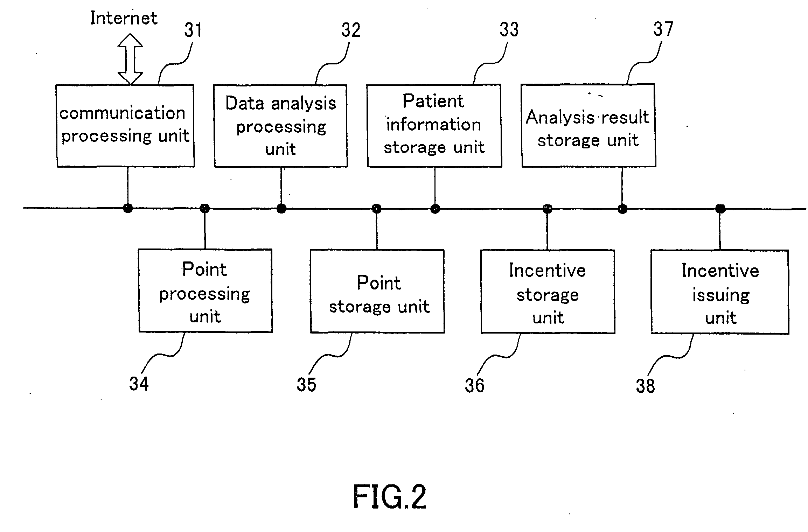

Hereinafter, the configuration of the self-measurement assisting server 6 will be described with reference to Figure 2.

As shown in Figure 2, the self-measurement assisting server 6 includes a communication processing unit 31, a data analysis processing unit 32, a patient information storage unit 33, a point processing unit 34, a point storage unit 35, an incentive storage unit 36 (electronic medium reward storage unit), an analysis result storage unit 37, and an incentive issuing unit 38 (reward issuing unit).

The communication processing unit 31 controls communications with the mobile phone 3, the computer 5, or the like via the Internet 4. The data analysis processing unit 32 performs analysis processing of the measurement data of the blood sugar meter 1, such as plotting a graph of the blood sugar level, so that a doctor of the medical institution easily can grasp fluctuations in blood sugar level or the like. The result of the analysis processing is expressed as a Web page employing HTML or the like, for example, and is stored in the analysis result storage unit 37 with a predetermined URL being assigned thereto. Therefore, by accessing this URL from the computer 5 of the medical institution, the doctor or the like can see the fluctuations in the blood sugar level of each patient on a browser.

The measurement patient information storage unit 33 stores various kinds of information on the patient who owns the blood sugar meter 1, such as a name, a contact address, a patient ID, and a device ID of the blood sugar meter. The point storage unit 35 stores obtained points for each patient. The point processing part 34 updates the obtained points for the patient stored in the point storage unit 35 every time the measurement data are transmitted from the patient, as described later. When the obtained points of the patient exceed a predetermined value, the incentive issuing unit 38 issues an electronic medium reward (on-line incentive) to the patient according to the obtained points.

The on-line incentive may be a program, data, or the like, which can be transmitted to the electronic device of the patient via a communication line, and is stored in the incentive storage unit 36. For example, in the case where the electronic device of the patient is a game machine, a game program that can be executed by the game machine, data relating to a character appearing in the game and an item such as a tool used in the game, or the like can be used as the on-line incentive. In the case where the electronic device of the patient is an MP3 player, the on-line incentive may be music data in MP3 format. Alternatively, in the case where the electronic device of the patient is a personal computer, the on-line incentive may be electronic cash, a coupon, or the like that can be used in payment for goods in on-line shopping on the Internet or the like.

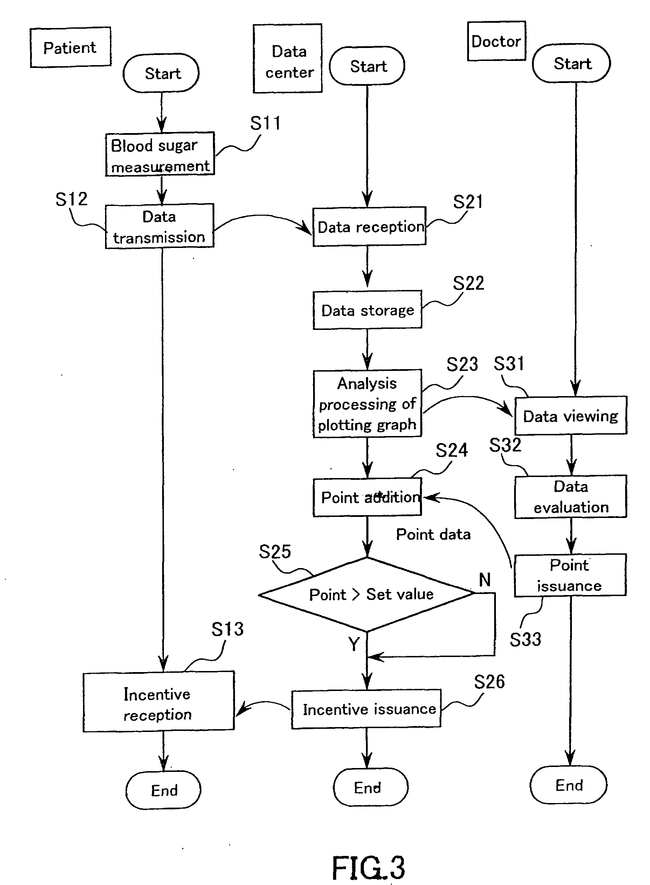

Hereinafter, the operation of this network system will be described with reference to the flowchart shown in Figure 3.

A patient performs a self-measurement of the blood sugar level using the blood sugar meter 1 in a predetermined manner (S11), and transmits the measurement data to the self-measurement assisting server 6 with a predetermined frequency, e.g., for each measurement, once a day, or the like (S12). The measurement data transmitted from the blood sugar meter 1 to the self-measurement assisting server 6 include the time at which the measurement was performed, a flag representing before or after a meal, a device ID or patient ID that will be described later, and the like, in addition to the measured blood sugar level.

As a manner of transmission of the measurement data from the blood sugar meter 1 to the self-measurement assisting server 6, there are at least the following two modes. A first mode is a data transmission via the game machine 2, as shown in Figure 1. Since the game machine 2 has the function of accessing the Internet 4 as described above, the measurement data can be transmitted from the blood sugar meter 1 to the game machine 2 via a connection cable 1a, and then transmitted to the self-measurement assisting server 6 via the game machine 2 and the mobile phone 3.

In this case, the device ID that is specific to the blood sugar meter 1 and is stored in the memory in the blood sugar meter 1 is transmitted from the blood sugar meter 1 to the game machine 2 along with the measurement data, and then transmitted to the self-measurement assisting server 6 along with the measurement data. The self-measurement assisting server 6 compares the received device ID with device IDs of respective patients stored in the patient information storage unit 33, thereby determining to which patient the received measurement data belong.

A second mode of the transmission of the measurement data from the blood sugar meter 1 to the self-measurement assisting server 6 is a data transmission in which the blood sugar meter 1 is connected to the mobile phone 3 without the intermediary of the game machine 2 to transmit the measurement data. In this case, the blood sugar meter 1 has the function of establishing a connection sufficient to enable data communication with the self-measurement assisting server 6 via the mobile phone 3, and transmits the measurement data with the device ID.

In either the first or second mode, a patient ID, which is previously stored in the blood sugar meter 1, may be used instead of or in addition to the device ID. Alternatively, instead of the device ID or the patient ID, the phone number or mail address of the mobile phone 3 may be used to identify the patient. To this end, the patient information storage unit 33 of the self-measurement assisting server 6 may store the device ID, the patient ID, or the phone number or mail address of the mobile phone 3 previously.

When the measurement data obtained by the blood sugar meter 1 are transmitted in this manner, the self-measurement assisting server 6 receives the transmitted measurement data (S21), and stores them in a temporary memory of the data analysis processing unit 32 in chronological order with respect to the device ID of the blood sugar meter 1 (S22).

On the basis of the stored measurement data, the data analysis processing unit 32 performs analysis processing such as plotting a graph that represents fluctuations in the blood sugar level of each patient (S23).

As shown in Figure 1, the computer 5 of the medical institution also is connected to this network system, and thus a doctor of the medical institution can view the measurement data of the self-measurement assisting server 6 and the result of the analysis processing thereof on the computer 5 (S31). For example, the data analysis processing unit 32 may provide the result of the above-described analysis processing in S23 as a Web page, and the doctor may view this Web page on the computer 5 by means of a WWW browser. In this case, in order to protect patient's privacy, it is preferable to limit the access to the Web page containing the result of the analysis processing of the measurement data so that people other than the doctor cannot access this page.

After viewing the result of the analysis processing of the measurement data on the computer 5, the doctor makes evaluations as to whether or not the measured blood sugar level is favorable, whether or not the measurement is performed properly in accordance with the previously given instructions as to time, the number of times, or the like (S32), and inputs points according to the evaluation result to the computer 5 (S33). At this time, the doctor can input his/her remarks on the measurement data to the computer 5 as a comment to the patient. The points and the comment input by the doctor are transmitted to the self-measurement assisting server 6 via the Internet 4.

In the self-measurement assisting server 6, the point storage unit 35 stores a cumulative total of points given by the doctor for respective patients, i.e., with respect to the device ID of the blood sugar meter 1. When the self-measurement assisting server 6 receives the points given by the doctor in S33 from the computer 5, the point processing unit 34 adds these points to the cumulative total of points of the patient stored in the point storage unit 35 (S24).

Then, the incentive issuing unit 38 compares the cumulative total of points updated in S24 with a previously set reference value (S25). When the cumulative total of points exceeds the reference value (Y as the result in S25), the incentive issuing unit 38 issues an incentive (S26). As an incentive, a character appearing in a game that is executed by the game machine 2, an item such as a tool used in the game, a game program, or the like may be used, for example.

When the incentive is issued, the patient connects the game machine 2 loaded with a cartridge storing a communication control program to the mobile phone 3, and accesses the self-measurement assisting server 6 via the Internet 4. The self-measurement assisting server 6 checks the connected game machine 2 for the connection ID (the patient ID or the device ID of the blood sugar meter 1). If the connected game machine 2 is identified as a recipient of incentive issuance, the self-measurement assisting server 6 transmits the incentive to the game machine 2. Thus, a game character, an item, or the like that has been issued as an incentive can be downloaded from the self-measurement assisting server 6 to the game machine 2 (S13).

Further, when the incentive is issued in Step S26, the patient may be allowed to select a desired incentive from among plural kinds of incentives within the range of obtained points. For example, data for showing a screen for incentive selection on the display of the game machine 2 is transmitted from the self-measurement assisting server 6 to the game machine 2 of the patient. Then, when the patient selects a desired incentive on the selection screen shown on the display of the game machine 2, data indicating the incentive selection made by the patient are transmitted to the self-measurement assisting server 6. On receipt of this data, in the self-measurement assisting server 6, the incentive issuing unit 38 transmits the incentive selected by the patient to the patient, and the point processing unit 34 subtracts the number of points corresponding to the incentive selected by the patient from the number of obtained points of the patient stored in the point storage unit 35.

Further, in Step S25, when the number of obtained points of the patient has not yet reached the value at which the incentive is issued, a message such as "Another ** points to get ****!" may be transmitted from the self-measurement assisting server 6 to the patient so that it is shown on the display of the mobile phone 3, the game machine 2, or the blood sugar meter 1. For diabetics, the original aim is to "successfully control the blood sugar level", and they are required to understand the significance thereof appropriately. However, it might be difficult for, for example, child diabetics to understand this significance. On this account, the present system is effective for a treatment for child diabetics because it provides a specific aim of getting a game character or the like by controlling the blood sugar level successfully, which is easy even for small children to understand.

The comment offered by the doctor in S32 is transmitted as appropriate from the self-measurement assisting server 6 to the mobile phone 3 of the patient, and is shown as appropriate on the display of the mobile phone 3, the game machine 2, or the blood sugar meter 1. This allows the patient to receive a brief diagnosis or guidance without visiting the medical institution.

As described above, according to the present embodiment, the number of points given by a doctor according to whether or not a patient performs a self-measurement at a given time, whether or not the measured blood sugar level is favorable, and the like is stored for respective patients in the self-measurement assisting server 6, and an on-line incentive such as a game character is given to the patient according to the number of obtained points.

Games of collecting various kinds of characters enjoy popularity among children not only in Japan but also around the world. Thus, when such game characters are supplied as on-line incentives, child diabetics strongly are motivated to perform a self-measurement in a predetermined manner every time and to try to follow the guidance of a doctor on food or exercise in an effort to control the blood sugar level successfully. Consequently, the system is effective in treatment of diabetes and prevention of complications associated with diabetes, and hence, it contributes to the whole society by saving on healthcare insurance costs.

Further, it becomes possible for doctors in medical institutions to grasp daily fluctuations in the blood sugar level of each patient, which allows them to give appropriate guidance to the patient. Furthermore, efficient examinations also become possible because the doctors can grasp a condition of a patient before the patient visits a medical institution. Moreover, by encouraging patients having the blood sugar meter 1 to use this system, each medical institution can gain patient loyalty.

### (Embodiment 2)

In above-described Embodiment 1, a doctor who accesses the self-measurement assisting server 6 to check the measurement data issues points. According to Embodiment 2, on the other hand, in a self-measurement assisting server 6, points are issued according to a predetermined standard when the received measurement data are subjected to analysis processing.

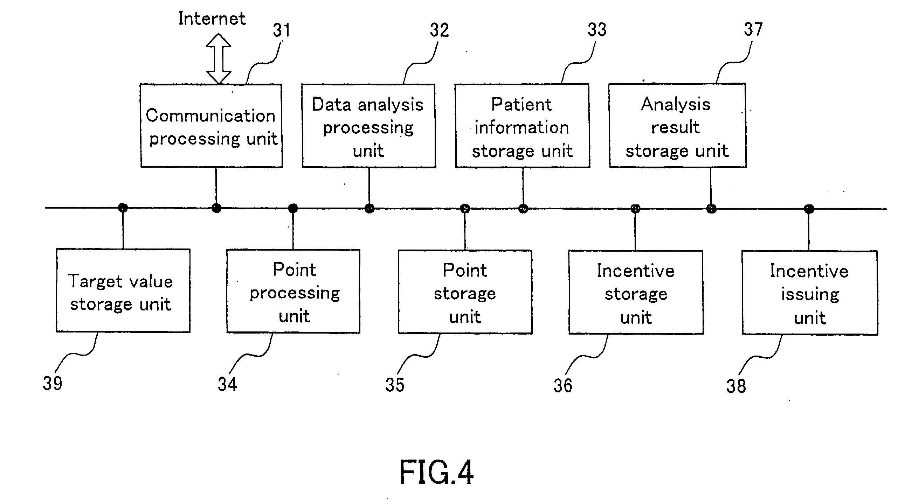

To this end, as shown in Figure 4, the self-measurement assisting server 6 according to Embodiment 2 includes a target value storage unit 39 in addition to the components as shown in Figure 2 for Embodiment 1. The target value storage unit 39 stores a target value set for each patient. A point processing part 34 determines points to give to the patient on the basis of the result of the processing of measurement data by a data analysis processing unit 32 and the target value stored in the target value storage unit 39, and adds the thus-determined points to the points of the patient stored in a point storage unit 35.

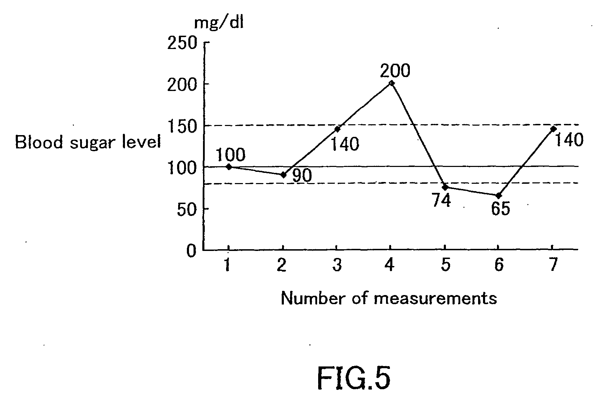

For example, in the case where a patient is directed by a doctor to control the blood sugar level within the range of 80-150 mg/dl, the doctor inputs from a computer 5 the target lower limit of the blood sugar level (80 mg/dl) and the target upper limit of the blood sugar level (150 mg/dl) for the patient. These values are transmitted to the self-measurement assisting server 6 and are stored in the target value storage unit 39. Here, if a measurement result of the patient in a certain day is as shown in Figure 5, the values obtained in four out of seven measurements in the day fall within the target range. Thus, the point processing unit 34 determines to give 4 points to the patient and adds 4 to the number of points of the patient stored in the point storage unit 35.

Further, as target values to be stored in the target value storage unit 39, various values can be used in addition to the above-described target lower and upper limits of the blood sugar level. While points are calculated based only on whether or not the blood sugar level is within the predetermined range in the above-described example, a plurality of conditions may be used in combination to determine points to be given.

For example, with regard to the control of the blood sugar level, a target central value may be defined in addition to the target lower and upper limits, and higher points may be given as the blood sugar level is closer to the target central value. For example, in the case where the target central value is 100 mg/dl, 3 points may be given when the blood sugar level is within the range of ± 10 mg/dl from the target central value, 2 points may be given when the blood sugar level is within the range of ± 20 mg/dl from the target central value excluding the above-described range, and 1 point may be given when the blood sugar level is within the range of 80-150 mg/dl excluding the above-described ranges.

Further, the number of points to be given preferably is determined according to whether or not measurements are performed regularly as directed by a doctor. For example, in the case where the doctor directs a patient to perform measurements four times a day at intervals of 3 hours or more, the number of measurements (4 times) and the minimum interval at which the measurements are performed (3 hours) are input to the computer 5 as target values, and these values are transmitted to the self-measurement assisting server 6 to be stored in the target value storage unit 39. Then, the point processing unit 34 receives the number and the interval of the measurements performed by the patient in a day from the data analysis processing unit 32 as the result of the processing of measurement data, and compares these values with the target values stored in the target value storage unit 39, thereby determining the number of points to give. For example, when both the number of measurements and the measurement interval achieve the targets, 2 points may be given, and when either one of them achieves the target, 1 point may be given.

Furthermore, a patient himself/herself may predict the blood sugar level before a measurement, and points may be given according to the degree of agreement between the predicted value and an actual measurement value. In this case, the patient inputs a predicted blood sugar level using an input button of a blood sugar meter 1 or a game machine 2, and thereafter performs a measurement of the blood sugar level with the blood sugar meter 1. The blood sugar meter 1 transmits both the predicted and actual measurement values of the blood sugar level to the self-measurement assisting server 6. The point processing unit 34 treats the predicted blood sugar level as a target value stored in the target value storage unit 39, calculates the difference between this value and the actual measurement value of the blood sugar level, and determines the number of points to give. For example, when the difference between the predicted and actual measurement values of the blood sugar level is within 10 mg/dl, 3 points may be given, and when the difference is within 20 mg/dl, 3 points may be given.

As described above, prediction of the blood sugar level before measurements has a dramatic effect on education of diabetics in that the prediction makes the patients recognize how their living conditions (food, exercise, and the like) affect fluctuations in blood sugar level. Further, as described above, by giving more points as the difference between the predicted and actual measurement values is smaller, the patients get more serious about predicting the blood sugar level, thereby making the above-described effect more remarkable.

The present invention is not restricted to the above-described respective embodiments, and various kinds of modifications can be made within the scope of the invention.

For example, as a measuring device, not only the above-described blood sugar meter but also an arbitrary measuring device can be used. Further, while the game machine has been taken as an example of an electronic device for receiving an on-line incentive, it is also possible to transmit a game, music data, or the like as an on-line incentive to a mobile phone as an electronic device, or to transmit an MP3 file to an MP3 player. Furthermore, the present invention also can be embodied by a single device incorporating a measuring device and an electronic device, such as a device having the functions of both the blood sugar meter and the game machine.

Further, while Figure 1 shows an example where the computer 5 of a medical institution is connected to the self-measurement assisting server 6 via the Internet 4, the self-measurement assisting server 6 may serve as a service provider of the Internet 4 for the computer 5.

Furthermore, while Figure 2 shows an example where the self-measurement assisting server 6 includes storage means such as the patient information storage unit 33, the analysis result storage unit 37, the point storage unit 35, and the incentive storage unit 36, these storage units may be provided in a storage device independent of the server or in an external device such as a data server.

In Embodiments 1 and 2, the present invention is embodied as a device. However, the invention also can be embodied as a program that makes a computer execute the operation of the self-measurement assisting server 6 as described in Embodiments 1 and 2. In this case, by making an arbitrary computer read the program stored on an arbitrary recording medium such as a CD-ROM, or downloading the program into the arbitrary computer by means of a communication medium, it becomes possible to operate the computer as the self-measurement assisting server 6.

### Industrial Applicability

As described above, according to the present invention, an administrative device connected to a measuring device for self-measurement via a communication line manages a remaining amount of consumables used in the measuring device, supplies the consumables, and corrects measurement data according to the condition of the consumables. Thus, the present invention can provide a self-measurement assisting system that reduces the burden imposed on users of the measuring device.

## Claims

1. A measurement assisting device to be connected to a measuring device and an electronic device of measurers via a communication medium, the device comprising:
a point storage unit for storing an obtained point for each measurer;
a point processing unit for adding a point given according to an evaluation of measurement data of a measurer received from the measuring device via the communication medium to the previously obtained points of the measurer stored in the point storage unit;
an electronic medium reward storage unit for storing an electronic medium reward to be transmitted to the electronic device of the measurer via the communication medium and used therein; and
a reward issuing unit for transmitting the electronic medium reward according to the obtained point of the measurer from the electronic medium reward storage unit to the electronic device of the measurer via the communication medium.

2. The measurement assisting device according to Claim 1, wherein the electronic medium reward is a game program, a game character or item, music data, image data, or electronic cash or a coupon that can be used by the measurer via the communication medium in payment to at least one trader.

3. The measurement assisting device according to Claim 1 or 2, which is to be connected to an information processing device of an administrative institution via the communication medium, the device further comprising:
a reception processing unit for receiving a point from the information processing device via the communication medium, the point being determined by an administrator in the administrative institution according to the evaluation of the measurement data and input to the information processing device.

4. The measurement assisting device according to Claim 1 or 2, further comprising a reference value storage unit for storing a standard for calculating points,
wherein the point processing unit evaluates the measurement data received from the measuring device via the communication medium according to the standard stored in the reference value storage unit and determines a point to give to the measurer according to the evaluation.

5. The measurement assisting device according to Claim 4, wherein the reference value storage unit stores a target measurement value, and the point processing unit gives higher points as an actual measurement value is closer to the target measurement value.

6. The measurement assisting device according to Claim 4, wherein the reference value storage unit stores a predicted value input by the measurer before a measurement, and the point processing unit gives higher points as an actual measurement value is closer to the predicted value.

7. The measurement assisting device according to Claim 4, wherein the reference value storage unit stores a target value relating to measurement performing conditions, and the point processing unit gives higher points as a value relating to actual measurement performing conditions is closer to the target value.

8. The measurement assisting device according to any one of Claims 1 to 7, wherein a blood sugar meter is used as the measuring device.

9. A measuring device to be connected to the measurement assisting device according to any one of Claims 1 to 8 via a communication medium and transmit measurement data to the measurement assisting device via the communication medium,
wherein a point is given according to an evaluation of the measurement data in the measurement assisting device, and an electronic medium reward to be used in an electronic device of a measurer is supplied according to a cumulative total of the point.

10. The measuring device according to Claim 9, having the function of measuring the blood sugar level.

11. The measuring device according to Claim 9 or 10, integrated with the electronic device.

12. A program to be read in a computer to be connected to a measuring device and an electronic device of measurers via a communication medium, the computer including a point storage unit for storing an obtained point for each measurer and an electronic medium reward storage unit for storing an electronic medium reward to be transmitted to the electronic device of the measurers via the communication medium and used therein,
the program making the computer execute:
a point processing of adding a point given according to an evaluation of measurement data of a measurer received from the measuring device via the communication medium to the previously obtained points of the measurer stored in the point storage unit; and
a reward issuance processing of transmitting the electronic medium reward according to the obtained point of the measurer from the electronic medium reward storage unit to the electronic device of the measurer via the communication medium.

13. The program according to Claim 12, wherein a game program, a game character or item, music data, image data, or electronic cash or a coupon that can be used by the measurer via the communication medium in payment to at least one trader is used as the electronic medium reward.

14. The program according to Claim 12 or 13,
wherein the computer is to be connected to an information processing device of an administrative institution via the communication medium,
the program further making the computer execute a processing of receiving a point from the information processing device via the communication medium, the point being determined by an administrator in the administrative institution according to the evaluation of the measurement data and input to the information processing device.

15. The program according to Claim 12 or 13,
wherein the computer further includes a reference value storage unit for storing a standard for calculating points, and
in the point processing, the measurement data received from the measuring device via the communication medium are evaluated according to the standard stored in the reference value storage unit, and a point to be given to the measurer is determined according to the evaluation.

16. The program according to Claim 15, wherein the reference value storage unit stores a target measurement value, and higher points are given as an actual measurement value is closer to the target value in the point processing.

17. The program according to Claim 16, wherein the reference value storage unit stores a predicted value input by the measurer before a measurement, and higher points are given as an actual measurement value is closer to the predicted value in the point processing.

18. The program according to Claim 15, wherein the reference value storage unit stores a target value relating to measurement performing conditions, and higher points are given as a value relating to actual measurement performing conditions is closer to the target value in the point processing.
